# EUROPEAN PATENT APPLICATION

(11) **EP 2 367 006 A1**
(43) Date of publication of application: **21.09.2011**
(21) Application number: 10173884.7
(22) Date of filing: 24.08.2010
(51) Int. Cl.: G01N 33/68

(54) **PLGF-based means and methods for diagnosing cardiac causes in acute inflammation**

(71) Applicant: Roche Diagnostics GmbH, 68305 Mannheim (DE); F. Hoffmann-La Roche AG, 4070 Basel (CH)
(72) Inventor: The designation of the inventor has not yet been filed
(74) Representative: Dick, Alexander

(57) **Abstract**

The present invention relates to the field of diagnostic measures. Specifically, it relates to a method for diagnosing a cardiac disease as a cause of a symptom of acute inflammation in a subject exhibiting said symptom comprising determining the amount of the biomarker P1GF in a sample of said subject; and comparing said amount to a reference amount whereby a cardiac disease is diagnosed as the cause of the symptom of acute inflammation in the said subject. Further encompassed are diagnostic devices and kits for carrying out the aforementioned methods.

## Description

The present invention relates to the field of diagnostic measures. Specifically, it relates to a method for diagnosing in a subject exhibiting a symptom of acute inflammation whether a cardiac disease is an acute inflammation-associated cardiac disease or an acute inflammation-independent cardiac disease comprising determining the amount of the biomarker P1GF in a sample of said subject and comparing said amount to a reference amount whereby it is diagnosed whether the cardiac disease is an acute inflammation-associated cardiac disease or an acute inflammation-independent cardiac disease. Further encompassed are diagnostic devices and kits for carrying out the aforementioned methods.

Fever is a frequent event and caused by infections, microbial toxins, mediators of inflammation and immune reactions. It is associated with the release of pyrogenic cytokines such as II 1, Il 6, TNF and interferons. These cytokines are responsible for the release of prostaglandin E 2 (PGE 2) in peripheral tissues.(Dinarello C.A., Porat R., in Principles of Internal Medicine 17. Ed p 117 ff). Most fevers are associated with self limited infections, such as common viral diseases and are, therefore, harmless. Thus, in most cases fever can be treated with antipyretics in order to achieve relief of symptoms. Antipyretics might, however, be contraindicated in patients with bacterial infections as they may mask inadequately treated bacterial infections. Specifically bacterial and fungal infections may become systemic and might progress to systemic inflammatory response syndrome (SIRS) which is characterized by hyper- or hypothermia, tachypnoe, tachycardia, leucocytosis or leucopenia.

SIRS associated with proven or suspected infection is called Sepsis. Sepsis can proceed to severe sepsis, septic shock, refractory septic shock or multi-organ dysfunction syndrome (MODS). The latter conditions are associated with organ dysfunction, e.g. the cardiovascular, the renal, the respiratory, the cerebral or the hematologic system. Attempts to improve survival in case of systemic infections, specifically sepsis have failed in the past. The major reason for the treatment failure was the clinically apparent development of organ dysfunction which could not be reversed by the treatment. Thus, it is important to identify individuals at risk of complications early to provide them with timely treatment (e.g. antibiotics). (Munford in Harrison Principles of Internal Medicine, 17, p.1695 ff). In fact, the time which elapsed until administration of an appropriate treatment is the primary determinant of mortality in patients with sepsis (Gaieski 2010, Crit Care Med 38 1045-1053).

Current methods to assess prognosis of a patient (preferably in the intensive care unit (ICU)) include the APACHE II Score as well as the SOFA Score. The APACHE II Score includes the assessment of temperature, mean blood pressure, heart rate, respiratory rate, arterial pH, oxygenation, serum sodium, serum potassium, hematocrit and white blood cell count. In addition age, chronic health status and the Glasgow coma Score is considered. Various studies indicate that the APACHE II Score is useful in predicting survival, survival rates have been shown to be higher in postoperative cases at the same APACHE II Score level. The APACHE II scoring system is not restricted to the ICU but can also be performed in the emergency room, this system is however time consuming as it cannot differentiate between different organ failures (Kress J.P., Hall J.B. in Harrison Principles of Internal Medicine p 1673 ff).

An alternative to the APACHE II Scoreing system is the SOFA Score. THE SOFA Score (Sepsis related organ failure Assessment) includes the determination of oxygenation (paO2/Fi2 (mmHg), platelet counts, bilirubin, blood pressure, Glasgow coma scale as well as kidney function (creatinine and urine volume/day) (Vincent et al, Intensive Care Med 1996, 22: 707 - 10). The SOFA Scoring System is also associated with prognosis of survival.

Disadvantage of both systems are that they are laborious and time consuming and might not pick up early changes/deterioration in organ function, which is of importance in the early recognition of complications and for treatment decisions (see above) or for the identification of patients at increased risk of complications (see above).

However, fever also often accompanies other diseases affecting the efficient function of the immune system, e.g., by weakening the organism in general. Such diseases include in particular cardiovascular disease, such as heart failure.

Placenta growth factor (PIGF) is a member of the VEGF family of growth factors, encoding a protein with an approximately 50 % identity to VEGF in the platelet derived growth factor (PDGF) like domain. In humans and in contrast to mice (where only P1GF 2 is present) P1GF exists in four isoforms (P1GF 1-4). (Maglione D. et al, Proc. Natl Acad Sci USA 1991 88: 9267 - 9271). Thus as shown for VEGF the translation of data obtained in mice into humans is questionable (Carmeliet P. Nature 2005, 438, 932 - 936) P1GF has been shown to be expressed in various tissues such as the heart, the lung, the thyroid gland and the skeletal muscle ( Autiero M. et al J. Thromb Haemostas 2003, 1: 1356 - 1370). So far, it was suggested that P1GF is essentially contributed by the inflammatory response (see, e.g., Yano 2006, J Exp Medicine 203(6): 1447-1458; Yano 2008, J Exp Medicine 205(11): 2623-2631; US2009/0197794) and, consequently, P1GF was classified as an inflammation parameter which directly correlates with the presence and strength of acute inflammation conditions such as SIRS or Sepsis. P1GF was also reported, in general, in connection with inflammation associated cardiovascular disease such as atherosclerosis (see WO 2009/101037). However, the actual stenosis accompanying atherosclerotic diseases such as coronary artery disease or peripheral artery disease are dependent on other inflammatory markers such as LPa or CRP (see WO 2010/003776).

In an emergency unit, it is often decisive to efficiently and reliably identify whether a symptom of an acute inflammation such as fever is associated with an acute inflammation and whether a cardiac diseases is associated with or independent of a potential acute inflammation in order to decide on suitable therapeutic measures.

The technical problem underlying the present invention can be seen as the provision of means and methods for reliably and efficiently determining in a subject exhibiting a symptom of acute inflammation whether a cardiac disease is an acute inflammation-associated cardiac disease or an acute inflammation-independent cardiac disease. The technical problem is solved by the embodiments characterized in the claims and herein below.

The present invention relates to a method for diagnosing in a subject exhibiting a symptom of acute inflammation whether a cardiac disease is an acute inflammation-associated cardiac disease or an acute inflammation-independent cardiac disease comprising:
a) determining the amount of the biomarker P1GF in a sample of said subject; and
b) comparing said amount to a reference amount whereby it is diagnosed whether the cardiac disease is an acute inflammation-associated cardiac disease or an acute inflammation-independent cardiac disease.

Preferably, it is diagnosed whether the cardiac disease is an acute inflammation-associated cardiac disease or an acute inflammation-independent cardiac disease by carrying out the further step of c) diagnosing whether the cardiac disease in the subject is an acute inflammation-associated cardiac disease or an acute inflammation-independent cardiac disease based on the result of the comparison carried out in step b).

The method of the present invention, preferably, is an ex vivo method. Moreover, it may comprise steps in addition to those explicitly mentioned above. For example, further steps may relate to sample pre-treatments or evaluation of the results obtained by the method. The method may be carried out manually or assisted by automation. Preferably, step (a) and/or (b) may in total or in part be assisted by automation, e.g., by a suitable robotic and sensory equipment for the determination in step (a) or a computer-implemented comparison and/or diagnosis based on said comparison in step (b).

The term "symptom of acute inflammation" as used herein refers to at least one symptom selected from a) white blood cell count (WBC) > about 12,000/ µ/L or < about 4000/µ/L, b) body temperature > about 38°C or < about 36°C, c) heart rate > about 90 beats/minute, d) a respiratory rate > about 20 breaths/minute or a partial pressure of CO2 of less than about 32 mm Hg, and e) more than 10% immature white blood cells among the counted white blood cells. Preferably, the term relates to fever (hyperthermia), i.e. a body temperature higher than about 38°C. Fever is, more preferably, characterized by a body temperature measured (i) in the rectum of at least 37.5-38.3°C (100-101°F), (ii) in the mouth (oral) of at least 37.7°C (99.9°F) or (iii) in the arm (axillary) or in the ear (otic) of at least 37.2°C (99.0°F). How to measure whether a subject exhibits fever is well known in the art.

The term "cardiac disease" as used herein refers to a disease affecting the function of the heart. Preferably encompassed are atherosclerotic diseases such as coronary artery disease or peripheral artery disease. Also encompassed is heart failure which may arise from atherosclerosis, in particular, from coronary artery disease. More preferably, the cardiac disease is coronary artery disease and/or heart failure. The symptoms accompanying said diseases are well known in the art and are described in standard text books of medicine such Stadmen or Pschyrembl.

The term "acute imflammation" refers to an acute systemic inflammation response in the subject. Said acute systemic inflammation may result from an infectious cause such as an expanding local infection which cannot be efficiently ameliorated due to an impaired function of the immune system in the subject or may be elicited by a non-infectious cause including trauma, bums, pancreatitis, ischemia and/or hemorrhage. Accordingly, acute systemic inflammation is characterized by a systemically reduced overall immune defense in the subject. The acute systemic inflammation may progress into the even more severe systemic inflammatory response syndrome (SIRS). SIRS as used in accordance with the present invention, preferably, encompasses SIRS as defined on the ACCP/SCCM Consensus Conference Definitions (1992/2003) (see e.g. American College of Chest Physicians/Society of Critical Care Medicine Consensus Conference: definitions for sepsis and organ failure and guidelines for the use of innovative therapies in sepsis. Crit Care Med. 1992 Jun;20(6):864-74)). Preferably, a patient is considered to suffer from SIRS, if the patient displays at least 2 symptoms of the following symptoms a) to e): a) white blood cell count (WBC) > about 12,000/ µ/L or < about 4000/µ/L, b) body temperature > about 38°C or < about 36°C, c) heart rate > about 90 beats/minute, d) a respiratory rate > about 20 breaths/minute or a partial pressure of CO2 of less than about 32 mm Hg, and e) more than 10% immature white blood cells among the counted white blood cells. The white blood cell count is usually determined by automated counting devices. As far as children are concerned, the consensus criteria for diagnosing SIRS in a child are disclosed in Goldstein et al. (Goldstein 2005, Pediatr. Crit Care Med 2005, 6(1),2- 8; see in particular Tables 2 and 3). An asymptomatic child patient in the sense of the present invention is a patient displaying less than 2, preferably less than 1 symptom of the ones described in Goldstein et al. (supra) which is incorporated herein by reference. Furthermore, in cases where SIRS may develop into sepsis if said disorder is accompanied by a proven or suspected microbial etiology, e.g., a systemic infection. An infection in the sense of the present invention preferably is a viral, fungus or bacterial infection, preferably a bacterial infection associated with bacteria selected from E coli, staphylococcus aureus, Klebsiella pneumoniae, Streptococci or Pseudomonas aeroginosa. The infection may as well be an infection by a fungus selected from Candida albicans, Candida tropicalis or Aspergillus fumigatus. An infection is diagnosed on the basis of assays and criteria generally known to the physician. Preferably, the infection is diagnosed on the basis of a bacterial culture assay, e.g., a culture medium inoculated with a sample from the patient, or based on molecular diagnostic methods. A fungus infection may, for example, be determined based on the generally known test assays such as Septifast. Sepsis may furthermore be determined by the aforementioned criteria for SIRS and at least two of the following criteria in addition: a) presence of leucocytes in a physiologically sterile body fluid; b) peritonitis or perforated viscus; c) pneumonia with focal opacification or petechiae, purpura, or purpura fulminans; d) bacteriemia. Further symptoms or particularly severe complications accompanying SIRS or sepsis are described in standard text books of medicine such as Stadmen or Pschyrembl. More preferably, the acute inflammation as used herein refers to SIRS and/or sepsis.

The cardiac disease referred to herein can be an acute inflammation-associated cardiac disease or an acute inflammation-independent cardiac disease. An "acute inflammation-associated cardiac disease" as referred to herein is a cardiac disease which accompanies acute inflammation. Preferably, it is either caused by acute inflammation or it causes the development or progression of acute inflammation. An "acute inflammation-independent cardiac disease" as referred to herein is a cardiac disease which merely occurs together with the said symptom of acute inflammation but which is not accompanying acute inflammation. Preferably, the said cardiac disease neither causes nor otherwise effects the development of an acute inflammation in a subject.

The term "subject" as used herein relates to animals, preferably mammals, and, more preferably, humans. The subject according to the present invention shall exhibit a symptom of acute inflammation as described elsewhere herein. The subject may or may not have a known history of cardiovascular diseases and, in particular, cardiac diseases. Preferably, the subject does not suffer from a tumor. Moreover, the subject shall, preferably, exhibit a physiological kidney function. Preferably, the said kidney function is determined by physiological creatinin levels or physiological glomerular filtration rate (GFR).

The term "diagnosing" as used herein means assessing whether a subject exhibiting a symptom of acute inflammation has a cardiac disease being an acute inflammation-associated cardiac disease or an acute inflammation-independent cardiac disease. As will be understood by those skilled in the art, such an assessment is usually not intended to be correct for 100% of the subjects to be diagnosed. The term, however, requires that the assessment is correct for a statistically significant portion of the subjects (e.g. a cohort in a cohort study). Whether a portion is statistically significant can be determined without further ado by the person skilled in the art using various well known statistic evaluation tools, e.g., determination of confidence intervals, p-value determination, Student's t-test, Mann-Whitney test etc.. Details are found in Dowdy and Wearden, Statistics for Research, John Wiley & Sons, New York 1983. Preferred confidence intervals are at least 90%, at least 95%, at least 97%, at least 98% or at least 99 %. The p-values are, preferably, 0.1, 0.05, 0.01, 0.005, or 0.0001.

The term "sample" refers to a sample of a body fluid, to a sample of separated cells or to a sample from a tissue or an organ. Samples of body fluids can be obtained by well known techniques and include, preferably, samples of blood, plasma, serum, or urine, more preferably, samples of blood, plasma or serum. Tissue or organ samples may be obtained from any tissue or organ by, e.g., biopsy. Separated cells may be obtained from the body fluids or the tissues or organs by separating techniques such as centrifugation or cell sorting. Preferably, cell-, tissue or organ samples are obtained from those cells, tissues or organs which express or produce the peptides referred to herein.

The term "P1GF (Placental Growth Factor)" as used herein refers to a placenta derived growth factor which is a polypeptide having 149 amino acids in length and being highly homologous (53% identity) to the platelet-derived growth factor-like region of human vascular endothelial growth factor (VEGF). Like VEGF, P1GF has angiogenic activity in vitro and in vivo. For example, biochemical and functional characterization of P1GF derived from transfected COS-1 cells revealed that it is a glycosylated dimeric secreted protein which is able to stimulate endothelial cell growth in vitro (Maqlione1993, Oncogene 8(4):925-31). Preferably, P1GF refers to human P1GF, more preferably, to human P1GF having an amino acid sequence as shown in Genebank accession number P49763.2, GI: 17380553 (Genebank is available from the NCBI, USA under www.ncbi.nlm.nih.gov/entrez).Furthermore, the term encompasses variants of said specific human P1GF. Such variants have at least the same essential biological and immunological properties as the specific P1GF polypeptide. Variants are deemed to share the same essential biological and immunological properties if they are detectable by the same specific assays referred to in this specification, e.g., by ELISA assays using polyclonal or monoclonal antibodies specifically recognizing the said P1GF polypeptides. A preferred assay is described in the accompanying Examples. Moreover, it is to be understood that a variant as referred to in accordance with the present invention shall have an amino acid sequence which differs due to at least one amino acid substitution, deletion and/or addition wherein the amino acid sequence of the variant is still, preferably, at least 50%, 60%, 70%, 80%, 85%, 90%, 92%, 95%, 97%, 98%, or 99% identical with the amino sequence of the specific P1GF polypeptides. The degree of identity between two amino acid sequences can be determined by algorithms well known in the art. Preferably, the degree of identity is to be determined by comparing two optimally aligned sequences over a comparison window, where the fragment of amino acid sequence in the comparison window may comprise additions or deletions (e.g., gaps or overhangs) as compared to the reference sequence (which does not comprise additions or deletions) for optimal alignment. The percentage is calculated by determining the number of positions at which the identical amino acid residue occurs in both sequences to yield the number of matched positions, dividing the number of matched positions by the total number of positions in the window of comparison and multiplying the result by 100 to yield the percentage of sequence identity. Optimal alignment of sequences for comparison may be conducted by the local homology algorithm of Smith and Waterman Add. APL. Math. 2:482 (1981), by the homology alignment algorithm of Needleman and Wunsch J. Mol. Biol. 48:443 (1970), by the search for similarity method of Pearson and Lipman Proc. Natl. Acad Sci. (USA) 85: 2444 (1988), by computerized implementations of these algorithms (GAP, BESTFIT, BLAST, PASTA, and TFASTA in the Wisconsin Genetics Software Package, Genetics Computer Group (GCG), 575 Science Dr., Madison, WI), or by visual inspection. Given that two sequences have been identified for comparison, GAP and BESTFIT are preferably employed to determine their optimal alignment and, thus, the degree of identity. Preferably, the default values of 5.00 for gap weight and 0.30 for gap weight length are used. Variants referred to above may be allelic variants or any other species specific homologs, paralogs, or orthologs. Moreover, the variants referred to herein include fragments of the specific PLGF polypeptides or the aforementioned types of variants as long as these fragments have the essential immunological and biological properties as referred to above. Such fragments may be, e.g., degradation products or splice variants of the PLGF polypeptides. Further included are variants which differ due to posttranslational modifications such as phosphorylation or myristylation.

Determining the amount of P1GF or any other peptide or polypeptide referred to in this specification relates to measuring the amount or concentration, preferably, semi-quantitatively or quantitatively. Measuring can be done directly or indirectly. Direct measuring relates to measuring the amount or concentration of the peptide or polypeptide based on a signal which is obtained from the peptide or polypeptide itself and the intensity of which directly correlates with the number of molecules of the peptide present in the sample. Such a signal - sometimes referred to herein as intensity signal -may be obtained, e.g., by measuring an intensity value of a specific physical or chemical property of the peptide or polypeptide. Indirect measuring includes measuring of a signal obtained from a secondary component (i.e. a component not being the peptide or polypeptide itself) or a biological read out system, e.g., measurable cellular responses, ligands, labels, or enzymatic reaction products.

In accordance with the present invention, determining the amount of a peptide or polypeptide can be achieved by all known means for determining the amount of a peptide in a sample. Said means comprise immunoassay devices and methods which may utilize labeled molecules in various sandwich, competition, or other assay formats. Said assays will develop a signal which is indicative for the presence or absence of the peptide or polypeptide. Moreover, the signal strength can, preferably, be correlated directly or indirectly (e.g. reverse- proportional) to the amount of polypeptide present in a sample. Further suitable methods comprise measuring a physical or chemical property specific for the peptide or polypeptide such as its precise molecular mass or NMR spectrum. Said methods comprise, preferably, biosensors, optical devices coupled to immunoassays, biochips, analytical devices such as mass- spectrometers, NMR-analyzers, or chromatography devices. Further, methods include micro-plate ELISA-based methods, fully-automated or robotic immunoassays (available for example on Elecsys^{™} analyzers), CBA (an enzymatic Cobalt Binding Assay, available for example on Roche-Hitachi^{™} analyzers), and latex agglutination assays (available for example on Roche-Hitachi^{™} analyzers).

Preferably, determining the amount of a peptide or polypeptide comprises the steps of (a) contacting a cell capable of eliciting a cellular response the intensity of which is indicative of the amount of the peptide or polypeptide with the said peptide or polypeptide for an adequate period of time, (b) measuring the cellular response. For measuring cellular responses, the sample or processed sample is, preferably, added to a cell culture and an internal or external cellular response is measured. The cellular response may include the measurable expression of a reporter gene or the secretion of a substance, e.g. a peptide, polypeptide, or a small molecule. The expression or substance shall generate an intensity signal which correlates to the amount of the peptide or polypeptide.

Also preferably, determining the amount of a peptide or polypeptide comprises the step of measuring a specific intensity signal obtainable from the peptide or polypeptide in the sample. As described above, such a signal may be the signal intensity observed at an m/z variable specific for the peptide or polypeptide observed in mass spectra or a NMR spectrum specific for the peptide or polypeptide.

Determining the amount of a peptide or polypeptide may, preferably, comprises the steps of (a) contacting the peptide with a specific ligand, (b) (optionally) removing non-bound ligand, (c) measuring the amount of bound ligand. The bound ligand will generate an intensity signal. Binding according to the present invention includes both covalent and non-covalent binding. A ligand according to the present invention can be any compound, e.g., a peptide, polypeptide, nucleic acid, or small molecule, binding to the peptide or polypeptide described herein. Preferred ligands include antibodies, nucleic acids, peptides or polypeptides such as receptors or binding partners for the peptide or polypeptide and fragments thereof comprising the binding domains for the peptides, and aptamers, e.g. nucleic acid or peptide aptamers. Methods to prepare such ligands are well-known in the art. For example, identification and production of suitable antibodies or aptamers is also offered by commercial suppliers. The person skilled in the art is familiar with methods to develop derivatives of such ligands with higher affinity or specificity. For example, random mutations can be introduced into the nucleic acids, peptides or polypeptides. These derivatives can then be tested for binding according to screening procedures known in the art, e.g. phage display. Antibodies as referred to herein include both polyclonal and monoclonal antibodies, as well as fragments thereof, such as Fv, Fab and F(ab)₂ fragments that are capable of binding antigen or hapten. The present invention also includes single chain antibodies and humanized hybrid antibodies wherein amino acid sequences of a non-human donor antibody exhibiting a desired antigen-specificity are combined with sequences of a human acceptor antibody. The donor sequences will usually include at least the antigen-binding amino acid residues of the donor but may comprise other structurally and/or functionally relevant amino acid residues of the donor antibody as well. Such hybrids can be prepared by several methods well known in the art. Preferably, the ligand or agent binds specifically to the peptide or polypeptide. Specific binding according to the present invention means that the ligand or agent should not bind substantially to ("cross-react" with) another peptide, polypeptide or substance present in the sample to be analyzed. Preferably, the specifically bound peptide or polypeptide should be bound with at least 3 times higher, more preferably at least 10 times higher and even more preferably at least 50 times higher affinity than any other relevant peptide or polypeptide. Non-specific binding may be tolerable, if it can still be distinguished and measured unequivocally, e.g. according to its size on a Western Blot, or by its relatively higher abundance in the sample. Binding of the ligand can be measured by any method known in the art. Preferably, said method is semi-quantitative or quantitative. Further suitable techniques for the determination of a polypeptide or peptide are described in the following.

First, binding of a ligand may be measured directly, e.g. by NMR or surface plasmon resonance. Second, if the ligand also serves as a substrate of an enzymatic activity of the peptide or polypeptide of interest, an enzymatic reaction product may be measured (e.g. the amount of a protease can be measured by measuring the amount of cleaved substrate, e.g. on a Western Blot). Alternatively, the ligand may exhibit enzymatic properties itself and the "ligand/peptide or polypeptide" complex or the ligand which was bound by the peptide or polypeptide, respectively, may be contacted with a suitable substrate allowing detection by the generation of an intensity signal. For measurement of enzymatic reaction products, preferably the amount of substrate is saturating. The substrate may also be labeled with a detectable lable prior to the reaction. Preferably, the sample is contacted with the substrate for an adequate period of time. An adequate period of time refers to the time necessary for an detectable, preferably measurable, amount of product to be produced. Instead of measuring the amount of product, the time necessary for appearance of a given (e.g. detectable) amount of product can be measured. Third, the ligand may be coupled covalently or non-covalently to a label allowing detection and measurement of the ligand. Labeling may be done by direct or indirect methods. Direct labeling involves coupling of the label directly (covalently or non-covalently) to the ligand. Indirect labeling involves binding (covalently or non-covalently) of a secondary ligand to the first ligand. The secondary ligand should specifically bind to the first ligand. Said secondary ligand may be coupled with a suitable label and/or be the target (receptor) of tertiary ligand binding to the secondary ligand. The use of secondary, tertiary or even higher order ligands is often used to increase the signal. Suitable secondary and higher order ligands may include antibodies, secondary antibodies, and the well-known streptavidin-biotin system (Vector Laboratories, Inc.). The ligand or substrate may also be "tagged" with one or more tags as known in the art. Such tags may then be targets for higher order ligands. Suitable tags include biotin, digoxygenin, His-Tag, Glutathion-S-Transferase, FLAG, GFP, myc-tag, influenza A virus haemagglutinin (HA), maltose binding protein, and the like. In the case of a peptide or polypeptide, the tag is preferably at the N-terminus and/or C-terminus. Suitable labels are any labels detectable by an appropriate detection method. Typical labels include gold particles, latex beads, acridan ester, luminol, ruthenium, enzymatically active labels, radioactive labels, magnetic labels ("e.g. magnetic beads", including paramagnetic and superparamagnetic labels), and fluorescent labels. Enzymatically active labels include e.g. horseradish peroxidase, alkaline phosphatase, beta-Galactosidase, Luciferase, and derivatives thereof. Suitable substrates for detection include di-amino-benzidine (DAB), 3,3'-5,5'-tetramethylbenzidine, NBT-BCIP (4-nitro blue tetrazolium chloride and 5-bromo-4-chloro-3-indolyl-phosphate, available as ready-made stock solution from Roche Diagnostics), CDP-Star™ (Amersham Biosciences), ECF™ (Amersham Biosciences). A suitable enzyme-substrate combination may result in a colored reaction product, fluorescence or chemoluminescence, which can be measured according to methods known in the art (e.g. using a light-sensitive film or a suitable camera system). As for measuring the enyzmatic reaction, the criteria given above apply analogously. Typical fluorescent labels include fluorescent proteins (such as GFP and its derivatives), Cy3, Cy5, Texas Red, Fluorescein, and the Alexa dyes (e.g. Alexa 568). Further fluorescent labels are available e.g. from Molecular Probes (Oregon). Also the use of quantum dots as fluorescent labels is contemplated. Typical radioactive labels include ³⁵S, ¹²⁵I, ³²P, ³³P and the like. A radioactive label can be detected by any method known and appropriate, e.g. a light-sensitive film or a phosphor imager. Suitable measurement methods according the present invention also include precipitation (particularly immunoprecipitation), electrochemiluminescence (electro-generated chemiluminescence), RIA (radioimmunoassay), ELISA (enzyme-linked immunosorbent assay), sandwich enzyme immune tests, electrochemiluminescence sandwich immunoassays (ECLIA), dissociation-enhanced lanthanide fluoro immuno assay (DELFIA), scintillation proximity assay (SPA), turbidimetry, nephelometry, latex-enhanced turbidimetry or nephelometry, or solid phase immune tests. Further methods known in the art (such as gel electrophoresis, 2D gel electrophoresis, SDS polyacrylamid gel electrophoresis (SDS-PAGE), Western Blotting, and mass spectrometry), can be used alone or in combination with labeling or other dectection methods as described above.

The amount of a peptide or polypeptide may be, also preferably, determined as follows: (a) contacting a solid support comprising a ligand for the peptide or polypeptide as specified above with a sample comprising the peptide or polypeptide and (b) measuring the amount peptide or polypeptide which is bound to the support. The ligand, preferably chosen from the group consisting of nucleic acids, peptides, polypeptides, antibodies and aptamers, is preferably present on a solid support in immobilized form. Materials for manufacturing solid supports are well known in the art and include, inter alia, commercially available column materials, polystyrene beads, latex beads, magnetic beads, colloid metal particles, glass and/or silicon chips and surfaces, nitrocellulose strips, membranes, sheets, duracytes, wells and walls of reaction trays, plastic tubes etc. The ligand or agent may be bound to many different carriers. Examples of well-known carriers include glass, polystyrene, polyvinyl chloride, polypropylene, polyethylene, polycarbonate, dextran, nylon, amyloses, natural and modified celluloses, polyacrylamides, agaroses, and magnetite. The nature of the carrier can be either soluble or insoluble for the purposes of the invention. Suitable methods for fixing/immobilizing said ligand are well known and include, but are not limited to ionic, hydrophobic, covalent interactions and the like. It is also contemplated to use "suspension arrays" as arrays according to the present invention (Nolan 2002, Trends Biotechnol. 20(1):9-12). In such suspension arrays, the carrier, e.g. a microbead or microsphere, is present in suspension. The array consists of different microbeads or microspheres, possibly labeled, carrying different ligands. Methods of producing such arrays, for example based on solid-phase chemistry and photo-labile protective groups, are generally known (US 5,744,305).

The term "amount" as used herein encompasses the absolute amount of a polypeptide or peptide, the relative amount or concentration of the said polypeptide or peptide as well as any value or parameter which correlates thereto or can be derived therefrom. Such values or parameters comprise intensity signal values from all specific physical or chemical properties obtained from the said peptides by direct measurements, e.g., intensity values in mass spectra or NMR spectra. Moreover, encompassed are all values or parameters which are obtained by indirect measurements specified elsewhere in this description, e.g., response levels determined from biological read out systems in response to the peptides or intensity signals obtained from specifically bound ligands. It is to be understood that values correlating to the aforementioned amounts or parameters can also be obtained by all standard mathematical operations.

The term "comparing" as used herein encompasses comparing the amount of the peptide or polypeptide comprised by the sample to be analyzed with an amount of a suitable reference source specified elsewhere in this description. It is to be understood that comparing as used herein refers to a comparison of corresponding parameters or values, e.g., an absolute amount is compared to an absolute reference amount while a concentration is compared to a reference concentration or an intensity signal obtained from a test sample is compared to the same type of intensity signal of a reference sample. The comparison referred to in step (b) of the method of the present invention may be carried out manually or computer assisted. For a computer assisted comparison, the value of the determined amount may be compared to values corresponding to suitable references which are stored in a database by a computer program. The computer program may further evaluate the result of the comparison, i.e. automatically provide the desired assessment in a suitable output format. Based on the comparison of the amount determined in step a) and the reference amount, it is possible to assess whether a cardiac disease is an acute inflammation-associated cardiac disease or an acute inflammation-independent cardiac disease. Therefore, the reference amount is to be chosen so that either a difference or a similarity in the compared amounts allows identifying those test subjects which belong into the group of subjects exhibiting a symptom of acute inflammation having either an acute inflammation-associated cardiac disease or an acute inflammation-independent cardiac disease. The method allows either excluding (rule-out) or identifying (rule-in) subject as a subject suffering from (i) an acute inflammation- associated cardiac disease or (ii) an acute inflammation independent disease.

Accordingly, the term "reference amount" as used herein refers to an amount which allows assessing whether a subject exhibiting a symptom of acute inflammation has a cardiac disease being the cause of the said symptom. Accordingly, the reference may be derived, in principle, from:
(I) a subject known to suffer from (i) an acute inflammation- associated cardiac disease or (ii) an acute inflammation independent disease or
(II) a subject known not to suffer from (i) an acute inflammation- associated cardiac disease or (ii) an acute inflammation independent disease.

Preferably, said reference amount is derived from a subject or a group of subjects known not to suffer from an acute inflammation-associated cardiac disease. More preferably, the said reference amount is derived from a subject or group of subjects suffering from an acute inflammation-independent cardiac disease. Even more preferably, said subject or group of subjects suffers from heart failure and/or a coronary artery disease but, preferably, is known not to suffer from peripheral artery disease. Moreover, the subject shall also not suffer from abnormal kidney function as assessed by creatinine values above the normal range or a GFR below 60 ml/min, chronic liver disorders, overt tumor disorders, and/or severe chronic inflammatory diseases (e.g., rheumatoid arthritis, Crohns disease).

Preferably the following is applied as a diagnostic algorithm, if a reference amount is used which is derived from a subject or group of subjects which are either known to suffer from an acute inflammation-independent cardiac disease or which are known not to suffer from an acute inflammation-associated cardiac disease, an essentially identical or decreased amount for the biomarker in the sample compared to the reference amount is indicative for a subject having an acute inflammation-independent cardiac disease while an increased amount for the biomarker in the sample compared to the reference amount is indicative for a subject having an acute inflammation-associated cardiac disease.

Also preferably the following can be applied as well as a diagnostic algorithm, if a reference amount is used which is derived from a subject or group of subjects which are either known to suffer from an acute inflammation-associated cardiac disease or which are known not to suffer from an acute inflammation-independent cardiac disease, an essentially identical or increased amount for the biomarker in the sample compared to the reference amount is indicative for a subject having an acute inflammation-associated cardiac disease while a decreased amount for the biomarker in the sample compared to the reference amount is indicative for a subject having an acute inflammation-independent cardiac disease.

It will be understood that the biomarker is increased in any event in comparison to the amount found in a healthy subject or group of healthy subjects. The term "healthy" in this context means that the subject shall not exhibit symptoms of either acute inflammation or a cardiac disease and, preferably, also no symptoms of other diseases as well. An amount found in a healthy subject or group thereof is, preferably, the upper limit of the normal physiological amount for a biomarker determined as average or mean in a healthy subject or group thereof.

The reference amount applicable for an individual subject may vary depending on various physiological parameters such as age, gender, or subpopulation, as well as on the means used for the determination of the polypeptide or peptide referred to herein. A suitable reference amount may be determined from a reference sample to be analyzed together, i.e. simultaneously or subsequently, with the test sample.

Reference amounts can be calculated for a cohort of subjects as specified above based on the average or mean values for a given biomarker by applying standard statistically methods. In particular, accuracy of a test such as a method aiming to diagnose an event, or not, is best described by its receiver-operating characteristics (ROC) (see especially Zweig 1993, Clin. Chem. 39:561-577). The ROC graph is a plot of all of the sensitivity/specificity pairs resulting from continuously varying the decision threshold over the entire range of data observed. The clinical performance of a diagnostic method depends on its accuracy, i.e. its ability to correctly allocate subjects to a certain prognosis or diagnosis. The ROC plot indicates the overlap between the two distributions by plotting the sensitivity versus 1-specificity for the complete range of thresholds suitable for making a distinction. On the γ-axis is sensitivity, or the true-positive fraction, which is defined as the ratio of number of true-positive test results to the product of number of true-positive and number of false-negative test results. This has also been referred to as positivity in the presence of a disease or condition. It is calculated solely from the affected subgroup. On the x-axis is the false-positive fraction, or 1-specificity, which is defined as the ratio of number of false-positive results to the product of number of true-negative and number of false-positive results. It is an index of specificity and is calculated entirely from the unaffected subgroup. Because the true- and false-positive fractions are calculated entirely separately, by using the test results from two different subgroups, the ROC plot is independent of the prevalence of the event in the cohort. Each point on the ROC plot represents a sensitivity/-specificity pair corresponding to a particular decision threshold. A test with perfect discrimination (no overlap in the two distributions of results) has an ROC plot that passes through the upper left corner, where the true-positive fraction is 1.0, or 100% (perfect sensitivity), and the false-positive fraction is 0 (perfect specificity). The theoretical plot for a test with no discrimination (identical distributions of results for the two groups) is a 45° diagonal line from the lower left corner to the upper right corner. Most plots fall in between these two extremes. If the ROC plot falls completely below the 45° diagonal, this is easily remedied by reversing the criterion for "positivity" from "greater than" to "less than" or vice versa. Qualitatively, the closer the plot is to the upper left corner, the higher the overall accuracy of the test. Dependent on a desired confidence interval, a threshold can be derived from the ROC curve allowing for the diagnosis or prediction for a given event with a proper balance of sensitivity and specificity, respectively. Accordingly, the reference to be used for the aforementioned method of the present invention, i.e. a threshold which allows to discriminate between subjects being at increased risk for mortality or those which have a normal risk among a cohort of subjects suffering from acute inflammation can be generated, preferably, by establishing a ROC for said cohort as described above and deriving a threshold amount therefrom. Dependent on a desired sensitivity and specificity for a diagnostic method, the ROC plot allows deriving suitable thresholds. It will be understood that an optimal sensitivity is desired for excluding a subject for being at increased risk (i.e. a rule out) whereas an optimal specificity is envisaged for a subject to be assessed as being at an increased risk (i.e. a rule in).

Preferably, the median values determined in Table 1 may be also used as a basis for establishing thresholds. Preferably, said threshold for P1GF for a subject having a acute inflammation-independent cardiac disease is within the range of the 25^{th} percentile to the 75^{th} percentile of the subjects suffering from peripheral artery disease, i.e. about 15.97 to about 19.79 pg/ml, more preferably within the range of the 25^{th} percentile to the 75^{th} percentile of the subjects suffering from coronary artery disease and/or heart failure, i.e. about 6.08 to about 15.88 pg/ml.

About in the context of the present invention means +/- 20%, +/- 10%, +/- 5%, +/- 2 % or +/-1% from the said values. This also takes into account usual deviations caused by measurement techniques and the like.

In principle, the present invention also relates to a method for diagnosing in a subject exhibiting a symptom of acute inflammation whether a cardiac disease is an acute inflammation-associated cardiac disease or an acute inflammation-independent cardiac disease, said method comprising diagnosing whether a cardiac disease is an acute inflammation-associated cardiac disease or an acute inflammation-independent cardiac disease based on a comparison of the amount of the biomarker P1GF in a sample of said subject to a reference.

Advantageously, it has been found in the studies underlying the present invention that the amount of P1GF in a body fluid such as blood, plasma or serum can serve as a biomarker allowing to diagnose whether a cardiac disease is an acute inflammation-associated cardiac disease or an acute inflammation-independent cardiac disease in a subject exhibiting symptoms known to also accompany acute inflammation conditions, in particular fever. It was found that P1GF below a certain threshold level originates essentially from an acute inflammation-independent cardiac disease rather than from an acute inflammation or an acute inflammation-associated cardiac disease although the patient may exhibit symptoms of acute inflammation such as fever as well (see accompanying Examples, below). Above the said threshold, the P1GF will be contributed to the measured P1GF level in blood from the inflammatory responses in connection with an acute inflammation and an acute-inflammation-associated cardiac disease, if any. So far, it was suggested that P1GF is essentially contributed by the inflammatory response (see, e.g., Yano 2006, J Exp Medicine 203(6): 1447-1458; Yano 2008, J Exp Medicine 205(11): 2623-2631; US2003/0197794) and, consequently, P1GF was classified as an inflammation parameter which directly correlates with the presence and strength acute inflammation conditions such as SIRS or Sepsis. However, the findings underlying the present invention show that this is not the case and that cardiac diseases which are present in the patients may as well be the cause of increased P1GF levels. This is, furthermore, supported by the fact that no strong correlation was observed between P1GF levels in blood and the well accepted scoring systems for acute inflammation conditions, the APACHE II Score and the SOFA Score, respectively (see accompanying Examples, below).

This discrimination between the causes of an alerting symptom of acute inflammation such as fever is important in emergency units where it must be decided immediately whether a patient requires a treatment of acute inflammation or treatment of a cardiac disease or a combined treatment.

Thanks to the present invention, it is possible to more reliably diagnose and treat subjects in particular in emergency situation where a fast and reliable diagnosis of the cause underlying an observed symptom is required. Moreover, the time consuming, expensive and cumbersome diagnostic measures such as scoring systems can be avoided when applying the method of the invention as an aid for diagnosis.

It is to be understood that the definitions and explanations of the terms made above and below apply accordingly for all embodiments described in this specification and the accompanying claims.

In a preferred embodiment of the method of the present invention, a cardiac troponin and/or a natriuretic peptide is/are determined as a further biomarker in step a).

The term "natriuretic peptide" comprises Atrial Natriuretic Peptide (ANP)-type and Brain Natriuretic Peptide (BNP)-type peptides and variants thereof having the same diagnostic potential (see e.g. Bonow, 1996, Circulation 93: 1946-1950). ANP-type peptides comprise pre-proANP, proANP, NT-proANP, and ANP. BNP-type peptides comprise pre-proBNP, proBNP, NT-proBNP, and BNP. The pre-pro peptide (134 amino acids in the case of pre-proBNP) comprises a short signal peptide, which is enzymatically cleaved off to release the pro peptide (108 amino acids in the case of proBNP). The pro peptide is further cleaved into an N-terminal pro peptide (NT-pro peptide, 76 amino acids in case of NT-proBNP) and the active hormone (32 amino acids in the case of BNP, 28 amino acids in the case of ANP). Preferred natriuretic peptides according to the present invention are NT-proANP, ANP, NT-proBNP, BNP, and variants thereof. ANP and BNP are the active hormones and have a shorter half-life than their respective inactive counterparts, NT-proANP and NT-proBNP. BNP is metabolised in the blood, whereas NT-proBNP circulates in the blood as an intact molecule and as such is eliminated renally. The in-vivo half-life of NTproBNP is 120 min longer than that of BNP, which is 20 min (Smith 2000, J Endocrinol. 167: 239-46.). Preanalytics are more robust with NT-proBNP allowing easy transportation of the sample to a central laboratory (Mueller 2004, Clin Chem Lab Med 42: 942-4.). Blood samples can be stored at room temperature for several days or may be mailed or shipped without recovery loss. In contrast, storage of BNP for 48 hours at room temperature or at 4° Celsius leads to a concentration loss of at least 20 % (Mueller loc.cit.; Wu 2004, Clin Chem 50: 867-73.). Therefore, depending on the time-course or properties of interest, either measurement of the active or the inactive forms of the natriuretic peptide can be advantageous. The most preferred natriuretic peptides according to the present invention are NT-proBNP and variants thereof. As briefly discussed above, the human NT-proBNP, as referred to in accordance with the present invention, is a polypeptide comprising, preferably, 76 amino acids in length corresponding to the N-terminal portion of the human NT-proBNP molecule. The structure of the human BNP and NT-proBNP has been described already in detail in the prior art, e.g., WO 02/089657, WO 02/083913 or Bonow loc. cit. Preferably, human NT-proBNP as used herein is human NT-proBNP as disclosed in EP 0 648 228 B1. These prior art documents are herewith incorporated by reference with respect to the specific sequences of NT-proBNP and variants thereof disclosed therein. The NT-proBNP referred to in accordance with the present invention further encompasses allelic and other variants of said specific sequence for human NT-proBNP discussed above. Specifically, envisaged are variant polypeptides which are on the amino acid level at least 60 % identical, more preferably at least 70 %, at least 80 %, at least 90 %, at least 95 %, at least 98% or at least 99 % identical, to human NT-proBNP. The degree of identity between two amino acid sequences, in principle, can be determined by algorithms well known in the art. Preferably, the degree of identity is to be determined by comparing two optimally aligned sequences over a comparison window, where the fragment of amino acid sequence in the comparison window may comprise additions or deletions (e.g., gaps or overhangs) as compared to the reference sequence (which does not comprise additions or deletions) for optimal alignment. The percentage is calculated by determining the number of positions at which the identical amino acid residue occurs in both sequences to yield the number of matched positions, dividing the number of matched positions by the total number of positions in the window of comparison and multiplying the result by 100 to yield the percentage of sequence identity. Optimal alignment of sequences for comparison may be conducted by the local homology algorithm of Smith 1981, Add. APL. Math. 2:482, by the homology alignment algorithm of Needleman 1970, J. Mol. Biol. 48:443, by the search for similarity method of Pearson 1988, Proc. Natl. Acad Sci. (USA) 85: 2444, by computerized implementations of these algorithms (GAP, BESTFIT, BLAST, PASTA, and TFASTA in the Wisconsin Genetics Software Package, Genetics Computer Group (GCG), 575 Science Dr., Madison, WI), or by visual inspection. Given that two sequences have been identified for comparison, GAP and BESTFIT are preferably employed to determine their optimal alignment and, thus, the degree of identity. Preferably, the default values of 5.00 for gap weight and 0.30 for gap weight length are used. Substantially similar and also envisaged are proteolytic degradation products which are still recognized by the diagnostic means or by ligands directed against the respective full-length peptide. Also encompassed are variant polypeptides having amino acid deletions, substitutions, and/or additions compared to the amino acid sequence of human NT-proBNP as long as the said polypeptides have NT-proBNP properties. NT-proBNP properties as referred to herein are immunological and/or biological properties. Preferably, the NT-proBNP variants have immunological properties (i.e. epitope composition) comparable to those of NT-proBNP. Thus, the variants shall be recognizable by the aforementioned means or ligands used for determination of the amount of the natriuretic peptides. Biological and/or immunological NT-proBNP properties can be detected by the assay described in Karl et al. (Karl 1999, Scand J Clin Invest 59:177-181), Yeo et al. (Yeo 2003, Clinica Chimica Acta 338:107-115). Variants also include posttranslationally modified peptides such as glycosylated or myristylated peptides. Further, a variant in accordance with the present invention is also a peptide or polypeptide which has been modified after collection of the sample, for example by covalent or non-covalent attachment of a label, particularly a radioactive or fluorescent label, to the peptide.

The term "cardiac troponin" refers to all troponin isoforms expressed in cells of the heart and, preferably, the subendocardial cells. These isoforms are well characterized in the art as described, e.g., in Anderson 1995, Circulation Research, vol. 76, no. 4: 681-686 and Ferrieres 1998, Clinical Chemistry, 44: 487-493. Preferably, cardiac troponin refers to troponin T and/or troponin I, and, most preferably, to troponin T. It is to be understood that isoforms of troponins may be determined in the method of the present invention together, i.e. simultaneously or sequentially, or individually, i.e. without determining the other isoform at all. Amino acid sequences for human troponin T and human troponin I are disclosed in Anderson, loc cit and Ferrieres 1998, Clinical Chemistry, 44: 487-493. The term "cardiac troponin" encompasses also variants of the aforementioned specific troponins, i.e., preferably, of tropoinin T or troponin I. Such variants have at least the same essential biological and immunological properties as the specific cardiac troponins. In particular, they share the same essential biological and immunological properties if they are detectable by the same specific assays referred to in this specification, e.g., by ELISA Assays using polyclonal or monoclonal antibodies specifically recognizing the said cardiac troponins. Moreover, it is to be understood that a variant as referred to in accordance with the present invention shall have an amino acid sequence which differs due to at least one amino acid substitution, deletion and/or addition wherein the amino acid sequence of the variant is still, preferably, at least 50%, 60%, 70%, 80%, 85%, 90%, 92%, 95%, 97%, 98%, or 99% identical with the amino sequence of the specific troponin. Variants may be allelic variants or any other species specific homologs, paralogs, or orthologs. Moreover, the variants referred to herein include fragments of the specific cardiac troponins or the aforementioned types of variants as long as these fragments have the essential immunological and biological properties as referred to above. Such fragments may be, e.g., degradation products of the troponins. Further included are variants which differ due to posttranslational modifications such as phosphorylation or myristylation.

Preferably, if a reference amount for the aforementioned biomarker(s) is used which is derived from a subject or group of subjects which are either known to suffer from an acute inflammation-independent cardiac disease or which are known not to suffer from an acute inflammation-associated cardiac disease, an essentially identical or decreased amount for the biomarker in the sample compared to the reference amount is indicative for a subject having an acute inflammation-independent cardiac disease while an increased amount for the biomarker(s) in the sample compared to the reference amount is indicative for a subject having an acute inflammation-associated cardiac disease.

Also preferably the following can be applied as well as a diagnostic algorithm, if a reference amount is used which is derived from a subject or group of subjects which are either known to suffer from an acute inflammation-associated cardiac disease or which are known not to suffer from an acute inflammation-independent cardiac disease, an essentially identical or increased amount for the biomarker(s) in the sample compared to the reference amount is indicative for a subject having an acute inflammation-associated cardiac disease while a decreased amount for the biomarker(s) in the sample compared to the reference amount is indicative for a subject having an acute inflammation-independent cardiac disease.

It will be understood that the biomarker is increased in any event in comparison to the amount found in a healthy subject or group of healthy subjects. The term "healthy" in this context means that the subject shall not exhibit symptoms of either acute inflammation or a cardiac disease and, preferably, also no symptoms of other diseases as well.

In particular, if an amount of P1GF which is indicative for an acute inflammation-associated cardiac disease has been determined, the diagnosis of the said cardiac disease can be further strengthened by determining further cardiac biomarkers such as a natriuretic peptide and/or a cardiac troponin.

In a further preferred embodiment of the method of the present invention, said method further comprises recommending a therapy for the cardiac disease.

The term "recommending" as used herein means establishing a proposal for a therapy which could be applied to the subject. However, it is to be understood that applying the actual therapy whatsoever is not comprised by the term.

The therapy to be recommended depends on the outcome of the diagnosis provided by the method of the present invention.

If an acute inflammation-independent cardiac disease has been diagnosed, a therapy for the said cardiac disease shall be applied pivotally. As set forth elsewhere, preferably, the cardiac disease is heart failure and, consequently, said therapy of a cardiac disease is, preferably, a heart failure. Suitable therapies against heart failure are well known in the art and include, e.g., surgical intervention such as catherization of coronary vessels or bypass surgery, or drug based therapies including administration of ACE inhibitors, angiotensin receptor blocker (AT1 antagonists), aldosterone antagonists, beta blockers such as Bisoprolol, Carvedilol, Metoprolol or Nebivolol.

If an acute inflammation-associated cardiac disease is, an anti-sepsis therapy is pivotally recommended. The term "anti-sepsis therapy" as used herein refers to therapeutic measures which aim to treat or ameliorate sepsis or symptoms accompanied therewith. The term includes-drug based therapies as well as general aspects of handling of the patients, e.g., monitoring and intensive care. Such a drug based therapy may include antibiotic treatment without further delay (Gaieski 2010, Crit Care Med 38(4): 1045-1053). In addition treatment with activated protein C requires consideration (Toussaint 2009, NEJM 361:2646-2652). Preferably, anti-sepsis therapy as used herein comprises administration of antibiotics, cortisone, hydrocortisone or complement proteins including activated protein C (commercially available under the tradename Xigris^{®}) and, preferably, ACE inhibitors, AT 1 blockers, and/or aldosternon antagonists, in addition. Also preferably, the anti-sepsis therapy can be combined in such a case with the heart failure therapy. Further intensive care measures may be considered in addition where appropriate, including dialysis, artificial respiration and the like.

In a further preferred embodiment of the method of the present invention, TGF13-1, BMP2 and/or BMP7 is determined in addition or, preferably, instead of P1GF.

The present invention also relates to the use of the biomarker P1GF or a detection agent that specifically binds thereto in a sample of a subject exhibiting a symptom of acute inflammation for diagnosing whether a cardiac disease is an acute inflammation-associated cardiac disease or an acute inflammation-independent cardiac disease in said subject.

The term "detection agent" as used herein refers to an agent which is capable of specifically recognizing and binding to P1GF present in a sample. Moreover, the said agent shall allow for direct or indirect detection of the complex formed by the said agent and the biomarker. Direct detection can be achieved by including into the agent a detectable label. Indirect labelling may be achieved by a further agent which specifically binds to the complex comprising the biomarker and the detection agent wherein the said further agent is than capable of generating a detectable signal. Suitable compounds which can be used as detection agents are well known in the art. Preferably, the detection agent is an antibody or aptamere which specifically binds to the biomarker.

The present invention also encompasses a device adapted for carrying out the method of the present invention comprising
a) an analyzing unit comprising a detection agent which specifically binds to P1GF, said unit being adapted for determining the amount of P1GF in a sample of a subject exhibiting a symptom of acute inflammation and, preferably, also comprising a detection agent which specifically binds to a cardiac troponin and/or a natriuretic peptide adapted for determining the amount of the said cardiac troponin and/or natriuretic peptide in a sample of the said subject; and
b) an evaluation unit for comparing the determined amount with a reference amount whereby it can be diagnosed whether a cardiac disease is an acute inflammation-associated cardiac disease or an acute inflammation independent cardiac disease, said unit comprising a database with reference amount values derived from a subject as defined before and a computer-implemented algorithm for carrying out a comparison as specified above.

The term "device" as used herein relates to a system comprising the aforementioned units operatively linked to each other as to allow the diagnosis or monitoring according to the methods of the invention. Preferred detection agents which can be used for the analyzing unit are disclosed elsewhere herein. The analyzing unit, preferably, comprises said detection agents in immobilized form on a solid support which is to be contacted to the sample comprising the biomarkers the amount of which is to be determined. Moreover, the analyzing unit can also comprise a detector which determines the amount of detection agent which is specifically bound to the biomarker(s). The determined amount can be transmitted to the evaluation unit. Said evaluation unit comprises a data processing element, such as a computer, with an implemented algorithm for carrying out a comparison between the determined amount and a suitable reference. Suitable references can be derived from samples of subjects to be used for the generation of reference amounts as described elsewhere herein above. The results may be given as output of parametric diagnostic raw data, preferably, as absolute or relative amounts. It is to be understood that these data will need interpretation by the clinician. However, also envisage are expert system devices wherein the output comprises processed diagnostic raw data the interpretation of which does not require a specialized clinician.

Furthermore, a kit adapted for carrying out the method of the present invention is contemplated, said kit comprising a detection agent for the biomarker P1GF and, preferably, for a cardiac troponin and/or a natriuretic peptide as well as instructions for carrying out the said method.

The term "kit" as used herein refers to a collection of the aforementioned components, preferably, provided in separately or within a single container. The container also comprises instructions for carrying out the method of the present invention. These instructions may be in the form of a manual or may be provided by a computer program code which is capable of carrying out the comparisons referred to in the methods of the present invention and to establish a diagnosis accordingly when implemented on a computer or a data processing device. The computer program code may be provided on a data storage medium or device such as a optical storage medium (e.g., a Compact Disc) or directly on a computer or data processing device. Moreover, the kit may, preferably, comprise standards for reference amounts as described elsewhere herein in detail.

The present invention also contemplates a method for diagnosing the strength of acute inflammation in a subject suffering from acute inflammation comprising the steps of:
(a) determining the amount of P1GF in a sample of said subject; and
(b) comparing the determined amount to a reference amount whereby the strength of the acute inflammation is diagnosed.

The aforementioned method, preferably, allows for an early classification of the strength of an acute inflammation. The term "strength" as referred to herein refers to acute inflammation which has, e.g., an accompanying cardiac disease, or not. The strength of acute inflammation can be classified, preferably, according to the APACHE II and/or Sofa Scoring systems described elsewhere herein. Suitable reference amounts can be obtained from samples of subjects suffering from acute inflammation of a defined strength. Identity or Differences will than indicate whether the investigated subject suffers from acute inflammation of the same strength or from less or more pronounced acute inflammation.

It has been found in the studies underlying the present invention that the strength of acute inflammation in a subject which has been identified to indeed suffer from the said acute inflammation is reflected by the P1GF level measured in a sample of the said subject. In more sever stages of the acute inflammation where an acute inflammation-associated cardiac disease becomes more prominent, the P1GF level will be also affected by the cardiac disease and, thus, will, in principle, be higher than in less pronounced stages of the acute inflammation with less or without a cardiac disease.

Moreover, the present invention also contemplates a method for monitoring whether an anti-sepsis or heart failure therapy is successful in a subject comprising
(a) determining the amount of P1GF in a first sample of the subject and a second sample of the subject, wherein said first sample has been obtained prior and said second sample has been obtained after the onset of the anti-sepsis or heart failure therapy; and
(b) comparing the second to the first sample wherein a decrease of the P1GF determined in the second sample compared to the first sample is indicative for a therapy being successful.

The present invention also relates to method for recommending an anti-sepsis therapy based on the P1GF amount determined in a sample of a subject suffering from acute inflammation.

The present invention also relates, in general, to a method of diagnosing whether a subject exhibiting a symptom of acute inflammation is at increased risk of mortality comprising:
(a) determing the amount of P1GF in a sample of said subject; and
(b) comparing said amount to a reference amount whereby it is diagnosed whether the subject is at increased risk of mortality, or not.

The term "increased risk of mortality" as used herein refers to statistically significant increase in the likelihood for mortality for a subject compared to the average or mean mortality likelihood within a given cohort of subjects, e.g., subjects suffering from acute inflammation in the present case. Whether there is a statistically significant increase of the risk can be determined by the skilled artisan by applying statistical tests recited elsewhere herein. The risk is preferably assessed for a predictive window. Said window is preferably 30 days or less, more preferably up to 4 weeks, 3 weeks, 2 weeks, one week, 3 days, 2 days or 1 day.

Suitable reference amounts can be obtained from samples of subjects known to be at increased risk of mortality. Identical amounts will than indicate whether the investigated subject is also at increased risk or not. The same applies mutatis mutandis for references from subjects known not be at increased risk of mortality. A preferred reference amount is an amount of P1GF of about 41.44 pg/ml for survivors. A test amount being essentially identical or larger shall be indicative for a normal risk of mortality. Also, a preferred reference amount is an amout of P1GF of about 34.44 pg/ml for non-survivors. A test amount being essentially identical or lower shall be indicative for an increased risk of mortality.

Furthermore, the present invention also contemplates a method for diagnosing whether a subject exhibiting a symptom of acute inflammation is at increased risk of mortality comprising:
(a) determining the amount of (i) P1GF and (ii) a natriuretic peptide or a cardiac troponin in a sample of said subject;
(b) calculating a ratio of the amounts of (ii) the natriuretic peptide or the cardiac troponin and (i) P1GF; and
(c) comparing the calculated ratio of (b) with a reference ratio whereby it is diagnosed whether the said subject is at increased risk of mortality, or not.

The term "ratio" as used herein also encompasses all values which are derived from the ratios referred to above by standard mathematical operations. However, the said derived values shall correlate directly to the original ratios. Preferably, the ratio is either the ratio of the amount of a natriuretic peptide to the amount of P1GF or the amount of a cardiac troponin to the amount of P1GF.

The "reference ratios" are, preferably, the ratios of the median of the amounts of a natriuretic peptide to the median of the amounts of P1GF or the median of amounts of a cardiac troponin to the median of amounts of P1GF in either subjects exhibiting a symptom of acute inflammation known to survive or subjects exhibiting a symptom of acute inflammation known not to survive. Preferred reference ratios from survivors are for a natriuretic peptide/PlGF: about 6.1 to about 90.8, preferably about 23.0 and for a cardiac troponin/P1GF: about 0.187 to about 1.16, preferably, about 0.447. Preferred reference ratios from non-survivors are for a natriuretic peptide/P1GF: about 189.0 to about 1632.0, preferably about 378.0 and for a cardiac troponin/P1GF: about 0.784 to about 6.773, preferably, about 2.085.

If the calculated ratio of step (b) is essentially identical to a survivor reference ratio, this is indicative for a normal (i.e. non-increased risk for mortality). If the ratio is significantly increased with respect to the survivor reference ratio, this is indicative for an increased risk for mortality. If the calculated ratio of step (b) is essentially identical to a non-survivor reference ratio, this is indicative for an increased risk of mortality. If the ratio is significantly decreased with respect to the non- survivor reference ratio, this is indicative for a normal risk of mortality.

All references cited in this specification are herewith incorporated by reference with respect to their entire disclosure content and the disclosure content specifically mentioned in this specification.

### FIGURES

Figure 1: Weak correlation was observed at presentation between P1GF levels in blood and the results of the APACHE II Scoring.
Figure 2: Weak correlation was observed at presentation between P1GF levels in blood and the results of the SOFA Scoring.
Figure 3: Correlation analysis is illustrated between P1GF levels at presentation and NT-pro BNP and Troponin T levels at presentation for patient case 22. As can be seen P1GF levels did not correlate closely to NT-pro BNP and Troponin T levels.
Figure 4: Correlation analysis is illustrated between P1GF levels at presentation and NT-pro BNP and Troponin T levels at presentation for patient case 28. As can be seen P1GF levels did not correlate closely to NT-pro BNP and Troponin T levels.
Figure 5: Correlation analysis is illustrated between P1GF levels at presentation and NT-pro BNP and Troponin T levels at presentation for patient case 61. As can be seen P1GF levels did not correlate closely to NT-pro BNP and Troponin T levels.
Figure 6: Correlation analysis is illustrated between P1GF levels at presentation and NT-pro BNP and Troponin T levels at presentation for patient case 99. As can be seen P1GF levels did not correlate closely to NT-pro BNP and Troponin T levels.

### EXAMPLES

### Example 1: Determination of biomarkers P1GF, NT-proBNP and sensitive troponin T in emergency patients

NT-proBNP was determined with sandwich immuno-assays using COBAS-analyzers from Roche/Hitachi. The assays comprise two monoclonal antibodies specific for the respective peptide. The first of these iv biotinylated and the second one in labelled with a Tris(2,2'-bibyridyl)ruthemium (II)-complex. In a first incubation step both antibodies are incubated with the sample. A sandwich complex comprising the peptide to be determined and the two different antibodies is formed. In a next incubation step streptavidin-coated beads are added to this complex. The beads bind the sandwich complexes. The reaction mixture is then aspirated into a measuring cell where the beads are magnetically captured on the surface of the electrode. The application of a voltage then induces a chemiluminescent emission from the ruthenium complex which is measured by a photomultiplier. The emitted amount of light is dependent on the amount of sandwich complexes on the electrode. NT-proBNP amounts between 2 pg/ml and 35000 pg/ml can be measured.

P1GF was tested using an ELECSYS immunoassay which employs two antibodies that are specific for P1GF. The test can be carried out automatically using different Roche analysers including ELECSYS 2010 and cobra e411 and cobra e601. The test has a sensitivity of 3 pg/ml.

Troponin T (hsTNT) was also tested using Roche analysers, the test follows the same test principles as described for NT-pro BNP and P1GF. The high sensitivity Troponin T test used in this study has a sensitivity of 2 pg/ml and can be used on ELECSYS 2010 as well as on Cobas e411 an cobas e601 analysers.

A group of 212 patients presenting to the emergency department with a body temperature exceeding 38,5°C (degrees Celsius), a heart rate above 90 beats per minute and a ventilatory frequency above 20 beats per minute were included into the study. Patients on dialysis or with impaired kidney function were excluded from the study, at presentation in none of the patients acute kidney injury was diagnosed. They were 102 females (mean age 65 years (range 21 - 91 years) and 110 males, mean age 62 years (range 18 -94 years). Patients were followed 6, 24 and 72 hours after presentation. These time points (TP) are also termed TP 1 = at presentaion, TP 2, at 6 h follow up, TP 3 ( at 24 h follow up) und TP 4 ( at 72 hour follow up). In all patients the APACHE II Score and the SOFA Score were recorded, in addition death at 30 days.

A control group consisted of 149 clinically healthy individuals (n = 51 males, mean age 40 years (range 20 - 52 years); n = 98 females mean age 41 years (range 18 - 56 years)) were included into the study, they had repeatedly normal blood pressure, a normal electrocardiogram, no diabetes and no history of cardiac disease or other disorders that would have put them at increased risk of cardiac disorders.

Also a group of 239 patients with stable coronary artery disease served as a control, they had symptomatic but stable heart failure, a normal kidney function as measured by normal creatinine levels, they were 186 males and 53 females, mean age 61.2. years, ischemic heart disease was present in 212 patients, all others had non-ischemic heart disease.

A further control group consisted of 51 patients with clinically apparent peripheral artery disease of the lower limbs, they were 39 males and 12 females, mean age 59.3 years, all had normal kidney function. Median pain free walking distance was 120 meters (range 52 - 920 meters).

In yet a further additional cross-sectional study 132 patients presenting with pneumonia ( 83 males and 49 females), mean age 71 years and proven or suspected coronary artery disease were analysed. All patients presented with fever above 38.5 degrees Celsius. 8/132 patients with pneumonia had P1GF levels below the 75 % percentile of individuals with stable coronary artery disease.

In addition n=58 patients (41 males and 17 females) mean age 62 years with established chronic obstructive pulmonary disease (COPD) and suspected or proven coronary artery disease were tested. All patients presented with fever above 38.5 degrees Celsius and were clinically classified as exacerbation of COPD. Fifteen of the 58 patients tested had P1GF levels below the 75 % percentile of patient s with stable CAD.

As shown in Table 1 patients presenting to the emergency room with acute inflammation had significantly higher P1GF levels than patients suffering from heart failure (atherosclerosis in coronary artery disease or peripheral artery disease, "HF" and "PAD") or clinically healthy controls.

**Table 1: P1GF levels in healthy individuals, patients cardiac diseases (HF and PAD) and those exhibiting inflammation.**

| Percentiles | Healthy | HF | PAD | inflammation |
|---|---|---|---|---|
| | PIGF [pg/ml] | | | |
| 75 | 10.13 | 15.88 | 19.79 | 59.93 |
| 50 (Median) | 7.80 | 11.35 | 18.38 | 41.21 |
| 25 | 6.99 | 6.08 | 15.97 | 28.18 |

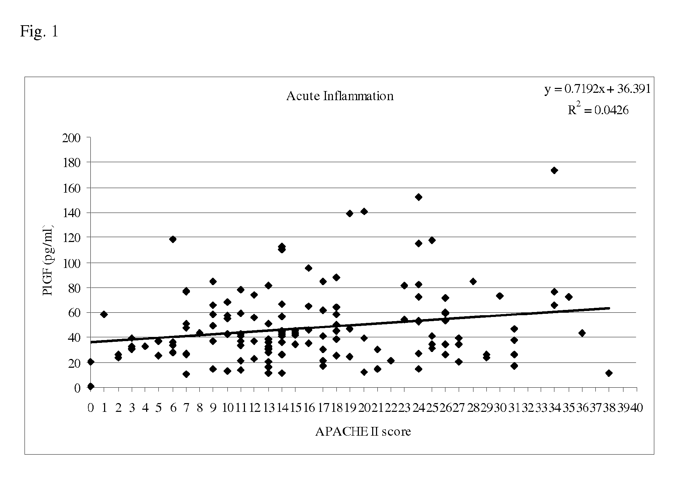

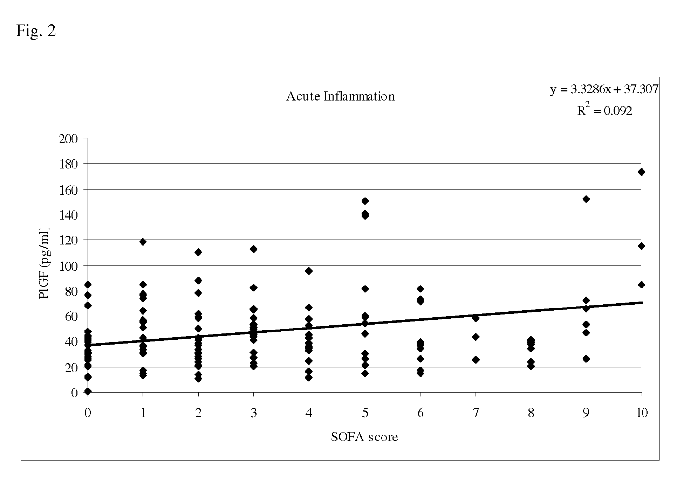

Moreover, when P1GF was correlated to the APACHE II and The SOFA Scores in a regression analysis, there was surprisingly only a very loose correlation of P1GF to both Scores. This is depicted in Figures 1 and 2. Accordingly, P1GF is apparently statistically independent of the the scoring results and, thus, does not pivotally reflect the inflammation status.

**Table 2: Correlation of P1GF above and below median und other test methods.**

| | P1GF | |
|---|---|---|
| | Below 41.21 pg/ml | Above 41.21 pg/ml |
| Troponin T [pg/ml] | 14.4 (5.5-52.3) | 27.0 (12.2 - 57.2) |
| NT-pro BNP [pg/ml] | 905.3 (185.0 - 4457.0) | 1436.0 (447.0 - 4533.0) |

The median is given for Troponin T and NT-pro BNP. The 25 und 75 percent percentile is shown in parenthesis.

Based on these results, it is apparent that a threshold value for P1GF can be used to identify patients which although exhibiting symptoms of acute inflammation in deed suffer from a cardiac disease rather than the said acute inflammation. A further cardiac biomarker like troponin T or NT-proBNP will further strengthen this diagnosis.

### Example 2: Ratios of P1GF and cardiac biomarkers are indicators of mortality risk

For the individuals of the aforementioned group of 212 patients presenting to the emergency department P1GF and other biomarkers were analyzed with respect to their potential as biomarkers for risk stratification. Specifically, ratios of the median values for P1GF and NT-proBNP or Troponin T in patients from which survived and those who died where calculated. Median values as well as percentiles are shown for survivors and non-survivors are shown in the following Tables 3 and 4:

**Table 3: Survivors**

| **Survivors** | **hsTNT** **pg/ml** | **NT-** **proBNP pg/ml** | **PLGF** **pg/ml** |
|---|---|---|---|
| **n** | **161** | **163** | **161** |
| **perc(25)** | **8.475** | **218.631** | **28.868** |
| **Median** **perc(50)** | **17.867** | **949.4 5 7** | **41.443** |
| **perc(75)** | **51.313** | **3650.254** | **59.92 7** |

**Table 4: Death**

| **Lethal: YES** | **hsTNT** **pg/ml** | **NT-** **proBNP** **pg/ml** | **PLGF** **PLGF** **pg/ml** |
|---|---|---|---|
| **n** | **11** | **11** | **11** |
| **perc(25)** | **2 7.3 72** | **6552.726** | **24.065** |
| **Median perc(50)** | **83.090** | **20712.699** | **34.553** |
| **perc(75)** | **202.925** | **37428.367** | **45.618** |

Follow up of P1GF in patients with acute infection:

Patients with acute infection were tested at presentation and at 3 time points of follow for P1GF. Results are summarized in the following Table 5:

**Table 5: Follow up for patients with acute infections**

| Time point (Percentile) | P1GF [pg/ml] | NT-proBNP [pg/ml] | hsTNT [pg/ml] |
|---|---|---|---|
| TP 1 (25) | 28.12 | 240.00 | 9.0 |
| TP 1 (50) | 41.21 | 1132.00 | 19.0 |
| TP 1 (75) | 59.93 | 4533.00 | 54.0 |
| | | | |
| TP 2 (25) | 23.75 | 506.00 | 13.0 |
| TP 2 (50) | 29.78 | 1743.00 | 26.0 |
| TP 2 (75) | 78.80 | 6414.00 | 72.0 |
| | | | |
| TP 3 (25) | 23.78 | 534.00 | 8.0 |
| TP 3 (50) | 31.07 | 1713.00 | 22.0 |
| TP 3 (75) | 79.04 | 5010.00 | 57.0 |
| | | | |
| TP 4 (25) | 20.98 | 496.00 | 7.0 |
| TP 4 (50) | 29.39 | 1767.00 | 15.0 |
| TP 4 (75) | 69.76 | 5303.00 | 54.0 |
| | | | |

| | | | |
|---|---|---|---|
| TP 1= at presentation; TP 2 = 6h follow up; TP3 = 24 h follow up; TP 4 = 72 h follow up. | | | |

Date presented present the Median ( 50 % percentile) and the 25 and 75 percent percentile in parenthesis.

As can be derived from the Table 3 while P1GF declined in most cases on follow up, NT-pro BNP and Troponin T values increased indicating that cardiac involvement associated with acute infection occurred with delay after acute infection and tended to improve only at 72 h follow up. The high variability found in individual cases in displayed in individual follow up cases.

Case 22:

| Timepoint: | P1GF | Troponin T NT-pro | BNP |
|---|---|---|---|
| 1 | 84.7 | 12.0 | 132 |
| 2 | 26.6 | 14.1 | 513 |
| 3 | 26.0 | 9.8 | 366 |
| 4 | 21.8 | 6.4 | 68 |

Case 28:

| | | | |
|---|---|---|---|
| 1 | 141.1 | 131.8 | 4558 |
| 2 | 127 | 167 | 9113 |
| 3 | 26 | 131.4 | 38176 |

Case 61

| | | | |
|---|---|---|---|
| 1 | 117.6 | 15,8 | 1446 |
| 2 | 36 | 60.5 | 17250 |
| 3 | 15 | 109 | 9650 |
| 4 | 24 | 192 | 7845 |

Case 99

| | | | |
|---|---|---|---|
| 1 | 74.1 | 12.3 | 308 |
| 2 | 17.2 | 15.4 | 1269 |
| 3 | 27.3 | 8.4 | 564 |
| 4 | 25.5 | 3.4 | 308 |

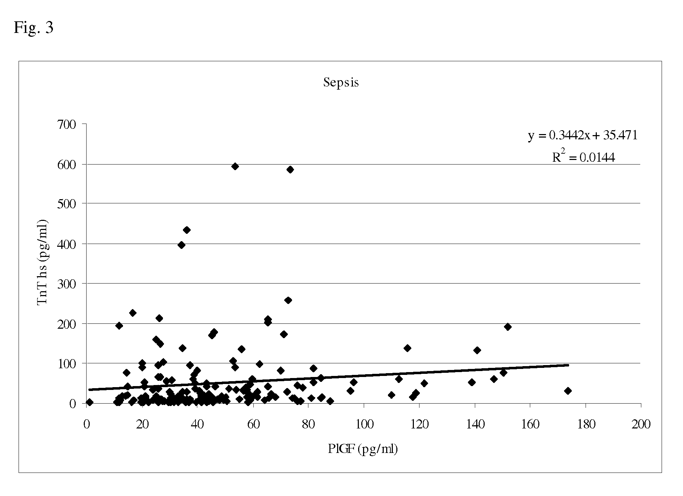

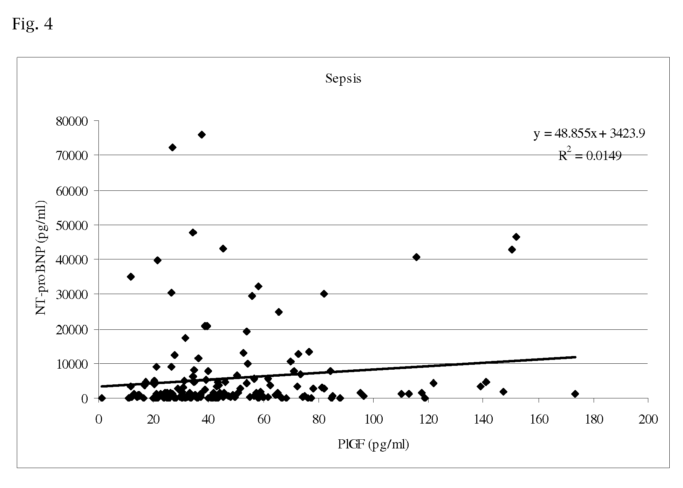

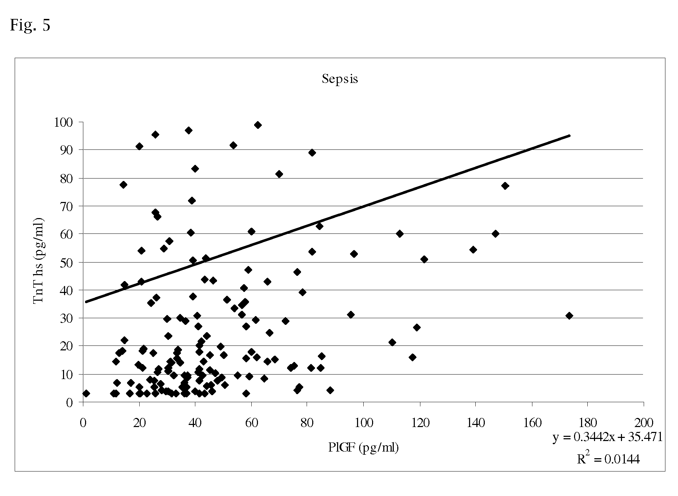

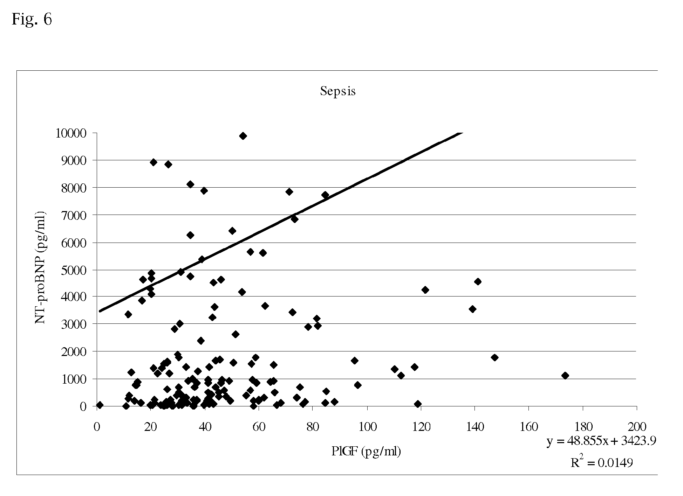

Figures 3 to 6 illustrate a correlation between P1GF levels at presentation and NT-pro BNP and Troponin T levels at presentation. As can be seen P1GF levels did not correlate closely to NT-pro BNP and Troponin T levels.

As can be seen from the cases shown above P1GF may preceed the development of cardiac damage or cardiac dysfunction and initially high P1GF levels should be regarded as a sign of "warning", similarly a decrease of P1GF is not associated with improved cardiac function.

Consistently elevated P1GF levels are indicators of lack of or delayed improvement as is shown in the cases to follow:

Case 144

| | | | |
|---|---|---|---|
| 1 | 81,9 | 88,9 | 30129 |
| 2 | 80,9 | 86,5 | 32170 |
| 3 | 77,9 | 96,5 | 37971 |
| 4 | 92,3 | 82,1 | 46277 |

Case 19

| | | | |
|---|---|---|---|
| 1 | 115,6 | 137 | 31030 |
| 2 | 108 | 122 | 35460 |
| 3 | 86 | 107 | 34254 |
| 4 | 84 | 95 | 21340 |

When patients were classified into survivors and those who died, it became apparent that survivors had an average P1GF value of 41,44 pg/ml and a median P1GF value of 41.443 pg/ml (see Table 3, above) and those who died had an average P1GF value of 34,44 pg/ml and a median P1GF value of 34.553 pg/ml (see Table 4, above) and, thus, a lower P1GF than those who survived. If combined with the information provided in the Table above this suggests that the inflammatory disease has more progressed in those who died than in those who survived.

This conclusion is further supported if P1GF results are combined with the results of NT-pro BNP and troponin T at presentation. Data were available from 161 survivors and from 11 patients who died.

Ratios of NT-proBNP/P1GF and Troponin T/PLGF, respectively, where calculated as follows:
Median Troponin T/Median P1GF

| | |
|---|---|
| = Survivors: | 0.447 (0.187; 1.16 ) |
| = Death: | 2.085 (0.784; 6.773) |

Median NT-pro BNP/Median P1GF

| | |
|---|---|
| = Survivors: | 23.0 (6.1; 90.8) |
| = Death: | 378.0 (189.0; 1632.0) |

Values are indicated as follows: Median (25th percentile; 75th percentile).

Survivors and non-survivors differ in the value of the ratios of either Troponin T to P1GF or NT-proBNP to P1GF. Moreover, the risk of mortality correlates with the said ratios. Specifically, low ratios are found in survivors while high ratios are found in non-survivors.

As can be seen from the table the NT-pro BNP/PIGF ratio appears to be superior to the Troponin T/PIGF ratio in terms of separation of survivors and those who died.

The data obtained can be explained by the fact that survivors were diagnosed earlier and also treated earlier and cardiac involvement associated with inflammation could be avoided or stopped in survivors and that cardiac involvement in patients who died was much to progressed to be treated adequately.

This study was conducted in patients presenting to the emergency room with symptoms severe enough to be candidates for hospital care. Patients tested included patients presenting with fever of any type as well as with pneumonia or chronic obstructive lung disorders. Using P1GF to discriminate underlying cardiac disorder from inflammation associated cardiac dysfunction only a minority of patients had evidence of underlying cardiac disorder. This is however different in outpatients who present with less severe symptoms and more frequently with viral infections. As cardiac and specifically cardiovascular disease is prevalent, in particular in the elderly outpatient population, determination of P1GF will be an efficient tool to discriminate underlying mere cardiac disease from an inflammation-associated cardiac disease, specifically if this test has been performed before an reference values are available. Thus the present invention will help to save antibiotics known to induce bacterial resistance in patients who do not deserve this therapy and also help to offer relief to patients in whom fever and symptom reducing drugs are not contraindicated.

## Claims

1. A method for diagnosing in a subject exhibiting a symptom of acute inflammation whether a cardiac disease is an acute inflammation-associated cardiac disease or an acute inflammation independent cardiac disease comprising:
a) determining the amount of the biomarker P1GF in a sample of said subject; and
b) comparing said amount to a reference amount whereby it is diagnosed whether the cardiac disease is an acute inflammation-associated cardiac disease or an acute inflammation-independent cardiac disease.

2. The method of claim 1, wherein said reference amount is derived from a subject or a group of subjects known to suffer from an acute inflammation-independent cardiac disease.

3. The method of claim 2, wherein said reference amount is derived from a subject or group of subjects also known to suffer from heart failure but not to suffer from peripheral artery disease.

4. The method of claim 2 or 3, wherein an essentially identical or decreased amount for the biomarker in the sample compared to the reference amount is indicative for a subject having an acute inflammation-independent cardiac disease.

5. The method of any one of claims 1 to 4, wherein a cardiac troponin and/or a natriuretic peptide is determined as a further biomarker in step a).

6. The method of claim 5, wherein, if the determined amount for P1GF is essentially identical or decreased in the sample compared to the reference amount, an increased amount for the said cardiac troponin and/or natriuretic peptide in the sample compared to the reference is furthermore indicative for a subject having an inflammation-independent cardiac disease.

7. The method of any one of claims 1 to 6 wherein said method further comprises recommending a therapy for the cardiac disease.

8. The method of claim 7, wherein said therapy of a cardiac disease is a heart failure therapy if the cardiac disease is an inflammation-independent cardiac disease.

9. The method claim 7, wherein said therapy of a cardiac disease is an anti-sepsis therapy if the cardiac disease is an inflammation-associated cardiac disease.

10. The method of claim 9, wherein said anti-sepsis therapy comprises administration of antibiotics, cortisone, hydrocortisone or complement proteins including activated protein C.

11. The method of any one of claims 1 to 10, wherein said cardiac disease is heart failure.

12. The method of any one of claims 1 to 11, wherein TGFβ-1 is determined instead of P1GF.

13. Use of the biomarker P1GF or a detection agent that specifically binds thereto in a sample of a subject exhibiting a symptom of acute inflammation for diagnosing whether a cardiac disease is an acute inflammation-associated cardiac disease or an acute inflammation independent cardiac disease.

14. A device adapted for carrying out the method of any one of claims 1 to 12 comprising
a) an analyzing unit comprising a detection agent which specifically binds to P1GF, said unit being adapted for determining the amount of P1GF in a sample of a subject exhibiting a symptom of acute inflammation and, preferably, also comprising a detection agent which specifically binds to a cardiac troponin and/or a natriuretic peptide adapted for determining the amount of the said cardiac troponin and/or natriuretic peptide in a sample of the said subject; and
b) an evaluation unit for comparing the determined amount with a reference amount whereby it can be diagnosed whether a cardiac disease is an acute inflammation-associated cardiac disease or an acute inflammation independent cardiac disease, said unit comprising a database with reference amount values derived from a subject as defined in claim 2 or 3 and a computer-implemented algorithm for carrying out a comparison as specified in claim 4 or 6.

15. A kit adapted for carrying out the method of any one of claims 1 to 12 comprising a detection agent for the biomarker P1GF and, preferably, for a cardiac troponin and/or a natriuretic peptide as well as instructions for carrying out the said method.
